# EUROPÄISCHE PATENTSCHRIFT

(11) **EP 0 970 944 B1**
(45) Veröffentlichungstag und Bekanntmachung des Hinweises auf die Patenterteilung: **27.03.2002**
(21) Anmeldenummer: 99110788.9
(22) Anmeldetag: 04.06.1999
(51) Int. Cl.: C07C 227/04, C07C 229/08

(54) **Verfahren zur Herstellung von Aminoessigsäureestern mit alpha-ständigem tertiären Kohlenwasserstoffrest**
Preparation of amino acetic acid esters substituted at position alpha by a tertiary hydrocarbon chain
Procédé de préparation d'esters d'acides aminoacétiques ayant en position alpha une chaîne carbonée tertiare

(30) Priorität: 09.07.1998 DE 19830632
(43) Veröffentlichungstag der Anmeldung: 12.01.2000
(73) Patentinhaber: Degussa AG, 40474 Düsseldorf (DE)
(72) Erfinder: Kleemiss, Wolfgang Dr., 45721 Haltern (DE)

(56) Entgegenhaltungen:
- DE-A- 19 724 086

## Beschreibung

Die Erfindung betrifft ein Verfahren zur Herstellung von Aminoessigsäureestern mit α-ständigem tertiären Kohlenwasserstoffrest der allgemeinen Formel I 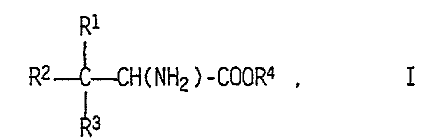 in der R¹, R² und R³ gleiche oder verschiedene Kohlenwasserstoffreste bedeuten, jeweils zwei dieser Reste mit dem Kohlenstoffatom. das sie substituieren, einen Kohlenstoffring bilden können und R⁴ einen Alkylrest mit 1 bis 4 Kohlenstoffatomen bedeutet,
aus den entsprechenden α-substituierten Malonsäuremonoamidestern der allgemeinen Formel II 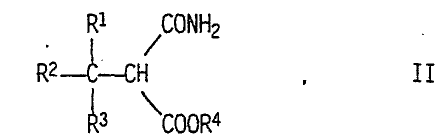 in der R¹, R², R³ und R⁴ die zuvor genannte Bedeutung haben.

Die einfachsten Verbindungen I sind die Methyl- oder Ethylester der α-tert.-Butylaminoessigsäure, die auch tert.-Leucin genannt wird (R¹, R² und R³ = Methyl, R⁴ = Methyl oder Ethyl).

Aminosäuren und deren Ester sind u.a. Komponenten für die Herstellung von Proteinen (siehe z.B. G.Krix, V.Eichhorn. H.-O.Jakubke, M.-R.Kula, Enzyme Microb. Technol., 21 [1997], 252). Aminosäuren mit α-ständigem tertiären Kohlenwasserstoffrest, wie tert.-Leucin sowie deren Ester, besitzen als nicht-proteinogene Eiweißbausteine eine erhebliche Bedeutung für die Synthese von biologisch aktiven Proteinen mit besonderer Wirkung (A.S.Bommarius, M.Schwarm. K.Stingl. M.Kottenhahn. K.Huthmacher und K.Drauz. Tetrahedron: Asymmetry 6 [1995], 2851). Tert.-Leucin dient weiterhin als Auxiliar für asymmetrische Synthesen (U.Schöllkopf, Pure and Applied Chem. 55 [1983]. 1799). Das zu diesen Zweck benötigte enantiomerenreine tert.-Leucin kann durch kinetische Racematspaltung von N-Acyltert.-leucinen mit Hilfe einer spezifischen Deacylase gewonnen werden (EP 494716).

Weiterhin läßt sich tert.-Leucin leicht in tert.-Leucinol umwandeln, das z.B. als chirales Auxiliar für die stereoselektive Synthese von Insektiziden verwendet wird (M.J.McKennon. A.I.Meyers. K.Drauz und M.Schwarm. J.Org.Chem. 58 [1993]. 3568). Aminoessigsäuren mit anderen tert.-Kohlenwasserstoffresten, d.h. höhere Homologe des tert.-Leucins, sollten in entsprechender Weise verwendbar sein.

Tert.-Leucin kann durch Strecker-Synthese aus Pivalinaldehyd (K.Ogura, Bull.Chem.Soc.Jpn. 65 [1992], 2359) oder durch Ammonolyse von 2-Brom-3,3-dimethylbuttersäure (E.Abderhalden. Z.Phys.Chem. 228 [1934], 193) hergestellt werden. Entsprechend lassen sich Aminoessigsäuren mit anderen α-ständigen tertiären Kohlenwasserstoffresten aus entsprechenden anderen Aldehyden durch Strecker-Synthese oder aus entsprechenden anderen Bromcarbonsäuren durch Ammonolyse herstellen. Ein weiteres bekanntes Herstellverfahren für tert.-Leucin ist die enzymkatalysierte Transaminierung von 3.3-Dimethyl-2-oxobuttersäure (EP 0248357).

Sowohl Pivalinaldehyd als auch 2-Brom-3,3-dimethylbuttersäure und 3,3-Dimethyl-2-oxobuttersäure sind relativ kostspielige Ausgangsstoffe. Das gilt auch für die erwähnten anderen Aldehyde und anderen Bromcarbonsäuren, aus denen Aminoessigsäuren mit anderen α-ständigen tert.-Kohlenwasserstoffresten hergestellt werden können. Für die Strecker-Synthese ist außerdem die sicherheitstechnisch anspruchsvolle Blausäure erforderlich. Bei der erwähnten enzymkatalysierten Transaminierung sind die Raum-Zeit-Ausbeuten unbefriedigend.

Weiterhin wurde die enantioselektive Synthese von (R)-tert.-Leucin beschrieben. Dabei wird aus tert.-Butylhydantoin mit Hilfe einer (R)-spezifischen Hydantoinase N-Carbamoyl-(R)-tert.-leucin gewonnen, das z.B. mit einer (R)-Carbamoylase in (R)-tert.-Leucin umgewandelt werden kann (DE 19529211). Man erhält (R)-tert.-Leucin in einer Ausbeute von 85,5%, bezogen auf das eingesetzte tert-Butylhydantoin, wobei jedoch in der Patentschrift keine Angaben über die Enantiomerenreinheit gemacht werden. Ferner wird in der Patentschrift die Spaltung des N-Carbamoyl-(R)-tert.-leucins mittels Nitrit beschrieben. Dabei ist die Reaktionszeit lang, und man erhält zudem ein wäßriges Reaktionsgemisch mit hohem Salzgehalt, das aufwendig durch Ionenaustauschchromatographie gereinigt werden muß.

Eine weitere Patentanmeldung (DE 19724086) beschreibt die Herstellung von Aminoessigsäuren mit α-ständigem tertiären Kohlenwasserstoffrest durch Hofmannschen Abbau von Malonsäuremonoamidestern mit α-ständigem tertiären Kohlenwasserstoffrest. Dabei wird nicht nur die Carbonamid-gruppe zur Aminogruppe abgebaut, sondern auch die Carbonestergruppe zur Carboxylgruppe verseift. In der Patentanmeldung wird auch die stereoselektive Synthese von (R)-tert.-Leucin aus (S)-tert.-Butylmalonsäuremonoamidester durch Hofmannschen Abbau beschrieben. Dabei werden 1,5 bis 4 Äquivalente Natronlauge und 1 bis 1,2 Äquivalente Natriumhypochlorit-Lösung eingesetzt. Man erhält die Aminoessigsäure in einer Ausbeute von 85%. Auch hier fällt die Aminoessigsäure nach der Neutralisation des Reaktionsgemisches mit Salzsäure zusammen mit großen Mengen Natriumchlorid als Koppelprodukt an, das wiederum mittels Ionenaustauschern abgetrennt wird.

Eine Aufgabe der Erfindung besteht darin, ein Verfahren bereitzustellen, nach dem man Ester von Aminoessigsäuren mit α-ständigem tertiären Kohlenwasserstoffrest, ausgehend von wohlfeilen Ausgangsstoffen mit guten Ausbeuten und Raum-Zeit-Ausbeuten sowie ohne Verwendung sicherheitstechnisch anspruchsvoller Einsatzstoffe und ohne eine aufwendige Abtrennung oder Reinigung, herstellen kann.

Erfindungsgemäß wird diese Aufgabe gelöst durch ein Verfahren zur Herstellung von Aminoessigsäureestern mit α-ständigem tertiären Kohlenwasserstoffrest der allgemeinen Formel I 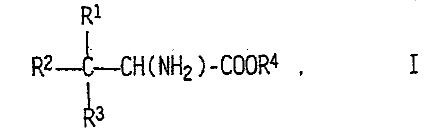 in der R¹, R² und R³ gleiche oder verschiedene Kohlenwasserstoffreste bedeuten, jeweils zwei dieser Rest mit dem Kohlenstoffatom, das sie substituieren, einen Kohlenstoffring bilden können und R⁴ einen Alkylrest mit 1 bis 4 Kohlenstoffatomen bedeutet,
aus den entsprechenden α-substituierten Malonsäuremonoamidestern der allgemeinen Formel II 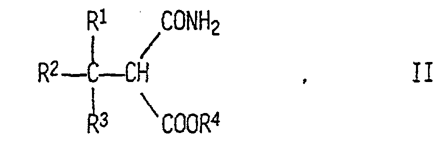 in der R¹, R², R³ und R⁴ die zuvor genannte Bedeutung haben, durch Hofmannschen Abbau mittels eines Hypohalogenits in wäßrig-basischem Milieu, wobei man das Hypohalogenit in Mengen von 1,0 bis 1,5 Äquivalenten und die Base in Mengen von 0,8 bis 1,5 Äquivalenten je Mol Edukt II anwendet.

Man erhält die Ester der Aminosäuren mit α-ständigem tertiären Kohlenwasserstoffrest trotz des vergleichsweise geringen Basenüberschusses in hohen Ausbeuten. Dies ist überraschend, denn der Hofmannsche Abbau wird zur Erzielung hoher Ausbeuten üblicherweise mit mindestens zwei Äquivalenten Base je Äquivalent Carbonamid-Gruppe durchgeführt (Organic Reactions III [1946], 2679). Weiterhin ist überraschend, daß die Carbonestergruppe unter den Bedingungen des Hofmann-Abbaus praktisch nicht angegriffen wird, obwohl Carbonsäureester üblicherweise mit verdünnten Laugen bei höherer Temperatur glatt zu den Carbonsäuresalzen verseift werden (siehe z.B. J.March, Advanced Organic Chemistry - Reactions, Mechanisms and Structure, 3rd Edition [1985], 334). Dies ist offenbar auf den tertiären Kohlenwasserstoffrest zurückzuführen. Im Gegensatz beispielsweise zum α-tert.-Butylmalonsäuremonoamidmethyl- oder -ethylester ergibt das entsprechende, nicht erfindungsgemäß einsetzbare n-Butyl-substituierte Isomere in erheblichen Mengen die entsprechende freie Säure bzw. deren Salz. Die praktische Unverseifbarkeit der Carbonestergruppe ist von besonderem Vorteil, weil sich das Reaktionsprodukt I als getrennte organische Phase aus dem wäßrigen Reaktionsgemisch abscheidet. Aufwendige Trennoperationen mittels Ionenaustauschern sind also nicht erforderlich. Wenn man die Ester gezielt sauer verseift, fallen die Aminosäuren zwar im Gemisch mit Salzen an. Die Salzmenge ist jedoch erheblich geringer als bei dem Verfahren nach der erwähnten DE 19724086.

Die Edukte II lassen sich in bekannter Weise mit hohen Gesamtausbeuten aus den entsprechenden tert-Alkylmalonsäuredialkylestern herstellen, die zunächst partiell zu den Monoestern verseift werden. Diese werden zum Carbonsäurehalogenid umgesetzt, das mit Ammoniak durch Reaktion mit Ammoniak das Monoamid II ergibt. (G.S.Bajwa, S.Chandrasekaran. J.H.Hargis, A.E.Sopchik, D.Blatter, W.G.Bentrude, J.Am.-Chem.Soc. 104 [1982], 6385). In der deutschen Patentanmeldung P 19623142.6 wird die Synthese von enantiomerenangereicherten tert.-Alkylmalonsäuremonoestern beschrieben. Diese können auf die beschriebene Weise in die entsprechenden enantiomerenangereicherten Edukte II überführt werden. In bevorzugten Edukten II (und damit auch in den Produkten I) stehen R¹, R² und R³ für einen Alkylrest mit 1 bis 4 Kohlenstoffatomen, einen Benzyl- oder Phenylethylrest oder bilden zwei dieser Substituenten mit dem Kohlenstoffatom, das sie substituieren, einen Cycloalkylring mit 5 bis 8 Ringgliedern. R⁴ bedeutet vorzugsweise Methyl oder Ethyl.

Alternativ kann man die Edukte II aus den entsprechenden tert.-Alkylmalonsäuremononitrilen durch Verseifung der Nitril- zur Carbonamidfunktion (Perez-Ossorio, Alemany, An.Soc.Espan. [B] 54, 471 [1958]) und anschließende Veresterung der Malonmonocarbonamidsäure herstellen.

Von den Edukten II seien im einzelnen beispielsweise genannt: α-tert.-Butylmalonsäuremonoamidmethylester, a-tert.-Butylmalonsäuremonoamidethylester, α-tert.-Pentylmalonsäuremonoamidethylester, α-(1-Methyl-1-phenylethyl)-malonsäuremonoamidethylester, α-[1'-Methylcyclohexyl-(1')]-malonsäuremonoamidethylester α-[2-Ethylbutyl-(2)]-malonsäuremonoamidethylester und α-[2-Benzylpropyl-(2)]-malonsäuremonoamidethylester.

Für den Hofmannschen Abbau werden eine Base und Hypohalogenit, d.h. ein Salz einer unterhalogenigen Säure, benötigt. Von den Hypohalogenitlösungen werden zweckmäßig die gut zugänglichen, wohlfeilen Hypochlorite eingesetzt. Die bevorzugten Hypochlorite sind Kaliumhypochlorit und insbesondere Natriumhypochlorit in Form einer wäßrigen Lösung, die auch als Bleichlauge bezeichnet wird. Calciumhypochlorit ist ebenfalls verwendbar, gibt aber geringere Ausbeuten. Es ist ein wesentliches Merkmal der Erfindung, daß das Hypohalogenit in den zuvor genannten Mengen verwendet wird. Es wird vorzugsweise in einer Menge von 1,0 bis 1,2 Äquivalenten, bezogen auf das Edukt II, angewandt.

Bevorzugte Basen sind die Alkalihydroxide, wie Kaliumhydroxid, und insbesondere Natriumhydroxid, wiederum in Form ihrer wäßrigen Lösungen. Auch Erdalkalihydroxide sind geeignet, ergeben aber geringere Ausbeuten. Im allgemeinen verwendet man eine Base mit dem Kation, das auch im Hypohalogenit vorliegt. Die zuvor angegebene Basenmenge ist kritisch und für den Verfahrenserfolg ausschlaggebend. Die Base wird vorzugsweise in einer Menge von 0,9 bis 1,2 Äquivalenten je Äquivalent des Edukts II eingesetzt.

Die Umsetzung findet in wäßrig basischem Milieu statt. Im allgemeinen entfallen auf das Wasser 50 bis 95, insbesondere 60 bis 90 Gew.-% des Reaktionsgemisches.

Das Verfahren nach der Erfindung kann kontinuierlich oder diskontinuierlich durchgeführt werden. Bei einer diskontinuierlichen Ausführungsform mit Natriumhydroxid als Base und Natriumhypochlorit als Salz einer unterhalogenigen Säure trägt man zunächst das Edukt II bei einer Temperatur, die im allgemeinen 0 bis 20°C. vorzugsweise 5 bis 10°C beträgt, in eine etwa 10 bis 15-gewichtsprozentige Natriumhypochlorit-Lösung ein. Man rührt das Gemisch bei dieser Temperatur etwa 1 bis 5 Stunden, vorzugsweise 2 bis 3 Stunden, gibt dann das Natriumhydroxid als wäßrige, in der Regel 5- bis 50-gewichtsprozentige Natronlauge zum Reaktionsgemisch und erwärmt auf eine Temperatur von 40 bis 100°C, vorzugsweise von 60 bis 80°C. Je nach der Temperatur ist die Reaktion nach 2 Minuten bis 3 Stunden, vorzugsweise nach 5 Minuten bis 2 Stunden beendet.

Nach dem Abkühlen des Reaktionsgemisches wird das Produkt I als obere, organische Phase abgetrennt. Die wäßrige, untere Phase kann man mit Hilfe eines inerten organischen Lösemittels extrahieren, um weiteres Produkt zu gewinnen. Die vereinigten organischen Phasen können nach dem Trocknen von organischem Lösemittel befreit werden. Man erhält so einen rohen Ester, der durch Destillation und/oder saure Extraktion mit anschließendem Alkalisieren des sauren Extraktes und Extraktion mit einem inerten organischen Lösemittel gereinigt werden kann. Man erhält so den Ester der entsprechenden Aminoessigsäure mit einer gaschromatographisch bestimmten Reinheit von >98% in einer Ausbeute von etwa 80%.

Wenn man ein enantiomerenangereichertes Edukt II wie beschrieben umsetzt. dann erhält man einen enantiomerenangereicherten Aminoessigsäureester I in gleicher Ausbeute und Reinheit unter Erhalt der Enantiomerenreinheit des Eduktes.

Die Reaktion kann auch in Gegenwart eines inerten organischen Lösemittels durchgeführt werden. Wenn dieses nicht mit dem wäßrigen Reaktionsmedium mischbar ist, wird der gebildete Aminoessigsäureester durch Extraktion dem Reaktionsmedium entzogen. Geeignete Lösemittel sind z.B. Alkohole, vorteilhaft mit 1 bis 4 Kohlenstoffatomen, wie Methanol, Ethanol und Isopropanol; Ether, beispielsweise mit 4 bis 10, vorteilhaft mit 4 bis 6 Kohlenstoffatomen, wie Diethylether und Methyl-tert.-butylether; sowie Kohlenwasserstoffe, vorteilhaft mit 5 bis 10 Kohlenstoffatomen, wie Toluol, Cyclohexan und aliphatische Kohlenwasserstoffe mit Siedepunkten zwischen 50 und 100°C.

Man kann das Verfahren nach der Erfindung auch kontinuierlich durchführen, z.B. analog der in EP 0 676 390 beschriebenen Arbeitsweise. Dazu wird ein Gemisch aus Edukt II kontinuierlich bei 0 bis 20°C, vorzugsweise bei 5 bis 10°C genügend lange mit einer Alkalihypohalogenit-Lösung in Berührung gebracht. Dann wird dem Reaktionsgemisch die Lösung eines Alkali- oder Erdalkalihydroxids zugefügt und das Reaktionsgemisch für 2 Minuten bis zu einer Stunde, vorzugsweise 2 bis 30 Minuten auf eine Temperatur von 50 bis 100°C erhitzt. Nach Beendigung der Reaktion wird das Reaktionsgemisch, wie beschrieben, kontinuierlich oder absatzweise aufgearbeitet. Die kontinuierliche Aufarbeitung schließt eine kontinuierliche Abtrennung des gebildeten Aminoessigsäureesters ein.

Der rohe oder gereinigte Aminoessigsäureester I kann in die freie Aminoessigsäure I umgewandelt werden, beispielsweise durch saure Verseifung, wie sie z.B, von D.A.Jaeger, M.D.Broadhurst. D.J. Cram in J.Am.Chem.Soc 101 (1979), 717 beschrieben wurde. Die Schutzwirkung des tertiären Kohlenwasserstoffrestes reicht offenbar nicht aus, um die Carbonestergruppe vor saurer Verseifung zu bewahren.

Die folgenden Beispiele sollen die Erfindung weiter erläutern, nicht aber ihren Schutzbereich begrenzen, wie er in den Patentansprüchen definiert ist.

### Beispiele

### Beispiel 1

In einem Rührgefäß werden 10 g (0.053 mol) tert.-Butylmalonsäuremonoethylesteramid in 36,9 g (0,058 mol) wäßriger 11.7-gewichtsprozentiger Natriumhypochloritlösung und 10 g Wasser bei 0 bis 5°C gerührt. Nach 3 h hat sich der Feststoff vollständig gelöst. Anschließend gibt man in der Kälte 10,6 g (0,053 mol) 20-gewichtsprozentige Natronlauge hinzu und erwärmt das Reaktionsgemisch rasch für 2,5 min auf 80°C. Die Reaktionstemperatur steigt dabei auf 103°C an. Man läßt das Reaktionsgemisch auf Raumtemperatur abkühlen und trennt die organische Phase ab. Die wäßrige Phase wird zweimal mit jeweils 100 ml Methyl-tert.-butylether extrahiert. Die vereinigten organischen Phasen werden über Natriumsulfat getrocknet und vom Lösemittel befreit. Mit Dioxan als internem Standard ergibt sich eine gaschromatographisch ermittelte Ausbeute an 2-tert.-Butyl-aminoessigsäureethylester (= tert.-Leucinethylester) von 6,32 g (75%).

### Beispiel 2

In einem Rührgefäß werden 10 g (0,053 mol) tert.-Butylmalonsäuremonoethylesteramid in 36,9 g (0,058 mol) wäßriger 11,7-gewichtsprozentiger Natriumhypochloritlösung und 10 g Wasser bei 0 bis 5°C gerührt. Nach 3 h hat sich der Feststoff vollständig gelöst. Anschließend gibt man in der Kälte 10,6 g (0,053 mol) 20-gewichtsprozentige Natronlauge hinzu und erwärmt das Reaktionsgemisch rasch für 2.5 min auf 80°C. Die Reaktionstemperatur steigt dabei auf 104°C an. Man läßt das Reaktionsgemisch auf Raumtemperatur abkühlen und trennt die organische Phase ab. Die wäßrige Phase wird zweimal mit jeweils 50 ml Methyl-tert.-butylether extrahiert. Die vereinigten organischen Phasen werden mehrmals mit 5-gewichtsprozentiger Salzsäure extrahiert. Danach werden die vereinigten sauren Extrakte mit verdünnter Natronlauge alkalisch gestellt und erneut mit Methyl-tert.-butylether extrahiert. Die organische Phase wird über Natriumsulfat getrocknet und vom Lösemittel befreit. Man erhält 6,2 g (74%) tert.-Leucinethylester mit einer durch GC ermittelten Reinheit von >98%.

### Beispiel 3

In einem Rührgefäß werden 30 g (0,16 mol) tert.-Butylmalonsäuremonoethylesteramid in 110,7 g (0,174 mol) wäßrige 11.7gewichtsprozentige Natriumhypochloritlösung und 30 g Wasser bei 0 bis 5°C gerührt. Nach 3 h hat sich der Feststoff vollständig gelöst. Anschließend gibt man in der Kälte 31,8 g (0,16 mol) 20-gewichtsprozentige Natronlauge hinzu und erwärmt das Reaktionsgemisch rasch für 2,5 min auf 80°C. Die Reaktionstemperatur steigt dabei auf 105°C an. Man läßt das Reaktionsgemisch auf Raumtemperatur abkühlen und trennt die organische Phase ab. Die wäßrige Phase wird zweimal mit jeweils 100 ml Methyl-tert.-butylether extrahiert. Die vereinigten organischen Phasen werden über Natriumsulfat getrocknet und vom Lösemittel befreit. Der Rückstand wird im Vakuum destilliert. Bei einem Druck von 6 mbar und einer Kopftemperatur von 75°C erhält man eine Hauptfraktion von 18.5 g. Diese enthält nach GC >98 % tert.-Leucinethylester. Die destillative Ausbeute beträgt also 79%.

### Beispiel 4

In einem Rührgefäß werden 10 g (0,053 mol) tert.-Butylmalonsäuremonoethylesteramid mit einem Enantiomerenüberschuß von 86% in 36,9 g (0.058 mol) wäßriger 11,7-gewichtsprozentiger Natriumhypochloritlösung und 10 g Wasser bei 0 bis 5°C gerührt. Nach 4 h hat sich der Feststoff vollständig gelöst. Anschließend gibt man in der Kälte 10.6 g (0,053 mol) 20-gewichtsprozentige Natronlauge hinzu und erwärmt das Reaktionsgemisch rasch für 3 min auf 80°C. Die Reaktionstemperatur steigt dabei auf 100°C an. Man läßt das Reaktionsgemisch auf Raumtemperatur abkühlen extrahiert das Reaktionsgemisch dreimal mit jeweils 50 ml Methyl-tert.-butylether. Die vereinigten organischen Phasen werden über Natriumsulfat getrocknet und vom Lösemittel befreit. Mit Dioxan als internem Standard ergibt sich eine gaschromatographisch ermittelte Ausbeute an tert.-Butylaminoessigsäureester von 5,93 g (70%).

Zur Bestimmung des Enatiomerenüberschusses wird das Reaktionsprodukt mit verdünnter Salzsäure extrahiert. Die saure wäßrige Phase wird mit verdünnter Natronlauge alkalisch gestellt und der Ethylester des tert.-Leucins mit Methyl-tert.-butylether extrahiert. Die organische Phase wird über Natriumsulfat getrocknet und vom Lösemittel befreit. Der Rückstand wird 2 h mit 20 g Ameisensäure unter Rückfluß erhitzt.

Nach Entfernung der Ameisensäure durch Destillation bleibt der N-Formyl-tert.-leucinethylester zurück. Die Analyse mit Hilfe einer chiralen GC-Säule ergibt einen Enatiomerenüberschuß des (R)-tert.-Leucinethylesters von 84%.

### Beispiel 5 (Vergleichsbeispiel)

In einem Rührgefäß werden 5 g (0,026 mol) n-Butylmalonsäuremonoethylesteramid in 16,27 g (0,028 mol) wäßriger 13-gewichtsprozentiger Natriumhypochloritlösung und 10 g Wasser bei 0 bis 5°C für 3 h gerührt. Danach hat sich der Feststoff vollständig gelöst. Anschließend gibt man in der Kälte 5,2 g (0.026 mol) 20-gewichtsprozentige Natronlauge hinzu und erwärmt das Reaktionsgemisch rasch für 2,5 min auf 80°C. Die Reaktionstemperatur steigt dabei auf 84°C an. Man läßt das Reaktionsgemisch auf Raumtemperatur abkühlen und extrahiert es dreimal mit jeweils 100 ml Methyl-tert.-butylether. Die vereinigten organischen Phasen werden über Natriumsulfat getrocknet und vom Lösemittel befreit. Als Rückstand verbleiben nur ca. 0,2 g an organischem Material, das keinen 2-Aminobuttersäureethylester enthält. Die bei der Reaktion entstandene 2-Aminobuttersäure verbleibt als Natriumsalz in der wäßrigen Phase. Der Ethylester kann also auf diese Weise nicht hergestellt werden.

## Patentansprüche

1. Verfahren zur Herstellung von Aminoessigsäureestern mit α-ständigem tertiären Kohlenwasserstoffrest der Formel I 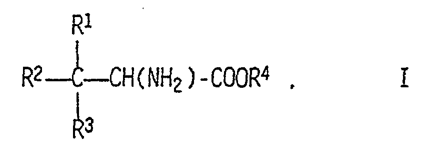 in der R¹, R² und R³ gleiche oder verschiedene Kohlenwasserstoffreste bedeuten, jeweils zwei dieser Rest mit dem Kohlenstoffatom. das sie substituieren, einen Kohlenstoffring bilden können und R⁴ Wasserstoff oder einen Alkylrest mit 1 bis 4 Kohlenstoffatomen bedeutet,
aus den entsprechenden α-substituierten Malonsäuremonoamidestern der Formel II 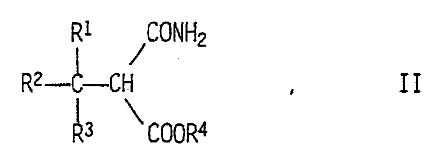 in der R¹, R², R³ und R⁴ die zuvor genannte Bedeutung haben. durch Hofmannschen Abbau mittels eines Hypohalogenits in wäßrig-basischem Milieu, **dadurch gekennzeichnet, daß** man das Hypohalogenit in Mengen von 1,0 bis 1,5 Äquivalenten und die Base in Mengen von 0,8 bis 1,5 Äquivalenten je Mol Edukt II anwendet.

2. Verfahren nach Anspruch 1, **dadurch gekennzeichnet, daß** man das Hypohalogenit in einer Menge von 1,0 bis 1,2 Äquivalenten und die Base in einer Menge von 0,9 bis 1,2 Äquivalente je Mol Edukt II anwendet.

3. Verfahren nach Anspruch 1 oder 2, **dadurch gekennzeichnet, daß** man als Hypohalogenit ein Alkalihypochlorit und als Base ein Alkalihydroxid verwendet.

4. Verfahren nach Anspruch 3, **dadurch gekennzeichnet, daß** das Alkalihypochlorit Natriumhypochlorit und das Alkalihydroxid Natriumhydroxid ist.

5. Verfahren nach einem der Ansprüche 1 bis 4, **dadurch gekennzeichnet, daß** man das Verfahren diskontiunierlich durchführt, indem man zunächst auf das Edukt II bei 0 bis 20°C 1 bis 5 Stunden lang eine Hypochlorit-Lösung einwirken läßt, dann die Base zusetzt, die Temperatur auf 40 bis 100°C steigert, die Reaktion 1 bis 5 Stunden fortsetzt und den gebildeten Aminoessigsäureester I als obere, organische Phase aus dem Reaktionsgemisch abtrennt.

6. Verfahren nach einem der Ansprüche 1 bis 4, **dadurch gekennzeichnet. daß** man das Verfahren kontinuierlich durchführt, indem man ein Gemisch aus Edukt II kontinuierlich bei 0 bis 20°C genügend lange mit einer Alkalihypohalogenit-Lösung in Berührung bringt, dem Reaktionsgemisch die Lösung eines Alkali- oder Erdalkalihydroxids zusetzt, das Reaktionsgemisch 2 min bis 1 h auf eine Temperatur von 50 bis 100°C erhitzt und es nach Beendigung der Reaktion kontinuierlich aufarbeitet, einschließlich einer kontinuierliche Abtrennung des gebildeten Aminoessigsäureesters I.

7. Verfahren nach einem der Ansprüche 1 bis 6, **dadurch gekennzeichnet, daß** man die Reaktion in Gegenwart eines inerten organischen Lösemittels durchführt.

8. Verfahren nach Anspruch 7, **dadurch gekennzeichnet, daß** man als Lösemittel einen Alkohol, einen Ether oder einen Kohlenwasserstoff verwendet.

## Claims

1. A process for the preparation of an aminoacetic acid ester with a tertiary hydrocarbon radical in the α-position, of the formula I 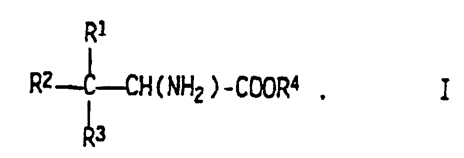 in which R¹, R² and R³ are identical or different hydrocarbon radicals, it being possible for in each case two of these radicals to form a carbon ring with the carbon atom on which they are a substituent and R⁴ is hydrogen or an alkyl radical having 1 to 4 carbon atoms,
from the corresponding α-substituted malonic acid monoamide ester of the formula II 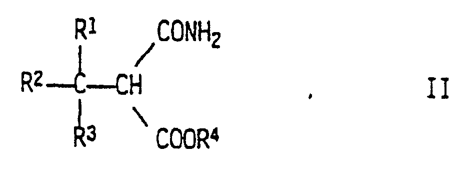 in which R¹, R², R³ and R⁴ have the abovementioned meanings,
by Hofmann degradation by means of a hypohalite in an aqueous-basic medium, **characterized in that** the hypohalite is used in amounts of from 1.0 to 1.5 equivalents and the base in amounts of from 0.8 to 1.5 equivalents per mole of starting material II.

2. A process according to claim 1, **characterized in that** the hypohalite is used in an amount of from 1.0 to 1.2 equivalents and the base is used in an amount of from 0.9 to 1.2 equivalents per mole of starting material II.

3. A process according to claim 1 or 2, **characterized in that** an alkali metal hypochlorite is used as the hypohalite and an alkali metal hydroxide is used as the base.

4. A process according to claim 3, **characterized in that** the alkali metal hypochlorite is sodium hypochlorite and the alkali metal hydroxide is sodium hydroxide.

5. A process according to any one of claims 1 to 4, **characterized in that** the process is carried out discontinuously by first allowing a hypochlorite solution to act on the starting material II at 0 to 20°C for 1 to 5 hours, then adding the base, increasing the temperature to 40 to 100°C, continuing the reaction for 1 to 5 hours and separating off from the reaction mixture, as the upper, organic phase, the aminoacetic acid ester I formed.

6. A process according to any one of claims 1 to 4, **characterized in that** the process is carried out continuously by bringing a mixture of starting material II into contact with an alkali metal hypohalite solution continuously at 0 to 20°C for a sufficiently long time, adding the solution of an alkali metal hydroxide or alkaline earth metal hydroxide to the reaction mixture, heating the reaction mixture at a temperature of 50 to 100°C for 2 minutes to 1 hour and, when the reaction has ended, working up the mixture continuously, including continuously separating off the aminoacetic acid ester I formed.

7. A process according to any one of claims 1 to 6, **characterized in that** the reaction is carried out in the presence of an inert organic solvent.

8. A process according to claim 7, **characterized in that** an alcohol, an ether or a hydrocarbon is used as the solvent.

## Revendications

1. Procédé de préparation d'esters d'acide aminoacétique ayant un reste hydrocarboné tertiaire situé en α, de formule 1, 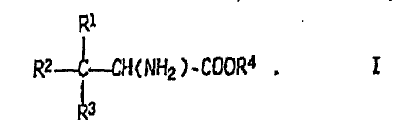 dans laquelle R¹, R² et R³ signifient des restes hydrocarboné identiques ou différents, respectivement deux de ces restes peuvent former avec l'atome de carbone qu'ils substituent, un cycle carboné et R⁴ signifie de l'hydrogène ou un reste alkyle ayant de 1 à 4 atomes de carbone,
à partir d'esters de monoamide d'acide malonique de formule II, 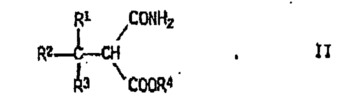 dans laquelle R¹, R² et R³ ont les significations mentionnées précédemment
par dégradation d'Hofmann à l'aide d'un hypohalogénite en milieu aqueux basique,
**caractérisé en ce qu'**
on utilise l'hypohalogénite en quantités de 1,0 à 1,5 équivalents et la base en quantités de 0,8 à 1,5 équivalents par mol d'éduit II.

2. Procédé selon la revendication 1,
**caractérisé en ce qu'**
on utilise l'hypohalogénite en une quantité allant de 1,0 à 1,2 équivalents et la base en une quantité de 0,9 à 1,2 équivalents, par mol d'éduit II.

3. Procédé selon l'une des revendications 1 ou 2,
**caractérisé en ce qu'**
on utilise comme hypohalogénite, un hypochlorite de métal alcalin et comme base un hydroxyde de métal alcalin.

4. Procédé selon la revendication 3,
**caractérisé en ce que**
l'hypochlorite de métal alcalin est l'hypochlorite de sodium et l'hydroxyde de métal alcalin est l'hydroxyde de sodium.

5. Procédé selon l'une quelconque des revendications 1 à 4,
**caractérisé en ce qu'**
on exécute le procédé d'une manière discontinue, procédé dans lequel on fait réagir en premier lieu une solution d'hypochlorite sur l'éduit II de 0 à 20°C pendant 1 à 5 heures, puis on ajoute la base, la température monte de 40 à 100°C, on poursuit la réaction pendant 1 à 5 heures et on sépare l'ester d'acide aminoacétique I formé en tant que phase supérieure organique, du mélange réactionnel.

6. Procédé selon l'une quelconque des revendications 1 à 4,
**caractérisé en ce qu'**
on exécute le procédé en continu, procédé dans lequel on met en contact un mélange à base d'éduit II, en continu de 0 à 20°C pendant suffisamment longtemps, avec une solution d'hypohalogénite de métal alcalin, on ajoute au mélange réactionnel la solution d'hydroxyde de métal alcalin ou de métal alcalino-terreux, on chauffe le mélange réactionnel pendant 2 minutes à 1 heure à une température de 50 à 100°C et on le soumet à un traitement après achèvement de la réaction, y compris à une séparation en continu, de l'ester d'acide aminoacétique I formé.

7. Procédé selon l'une quelconque des revendications 1 à 6,
**caractérisé en ce qu'**
on exécute la réaction en présence d'un solvant inerte.

8. Procédé selon la revendication 7,
**caractérisé en ce qu'**
on utilise comme solvant un alcool, un éther ou un hydrocarbure.
